# EUROPEAN PATENT APPLICATION

(11) **EP 1 923 468 A1**
(43) Date of publication of application: **21.05.2008**
(21) Application number: 06124262.4
(22) Date of filing: 16.11.2006
(51) Int. Cl.: C12N 15/867, A61K 39/12, C12Q 1/68

(54) **Lentiviral vectors for gene transfer in quiescent (G0) cells**

(71) Applicant: Bundesrepublik Deutschland, letztvertreten durch den Präsidenten des Paul-Ehrlich-Instituts Prof. Dr. Johannes Löwer, 63225 Langen (DE)
(72) Inventor: Cichutek, Klaus, 63225 Langen (DE); Schweizer, Matthias, 78115 Freiburg (DE); Schüle, Silke, 60596 Frankfurt (DE); Kloke, Björn-Philipp, 63225 Langen (DE)
(74) Representative: Grund, Martin

(57) **Abstract**

Vector particles of the present invention are derived from HIV-2 and further pseudotyped with a G protein from the VSV viral strain. Such vectors provide a means for efficiently transducing cells residing in the G0 phase of the cell cycle at a low MOI value. Thus the vector particles of the present invention can be readily utilized to safely and efficiently transduce quiescent cells including stem cells or precursor cells without the need to activate said cells by cell culture media and/or cytokines or growth factors. Thus, the vector particles of the present invention provide an improved means for applications in the fields of gene therapy, directed immunotherapy or vaccination, as HIV-2 is a human pathogen and, consequently, during treatment with pharmaceutical compositions based on the present vector particles, no additional foreign pathogen is thereby transferred to a human subject.

## Description

### Technical Field

The present invention relates to the pseudotyping of lentiviral vector particles having a heterologous envelope protein derived from vesicular stomatitis virus (VSV), and related uses of the vector particles derived therefrom.

### Background Art

Compared to known gene transfer systems, retroviral vectors offer a wide range of advantages, including their ability to transduce a variety of cell types, to stably integrate transferred genetic material into the genome of the targeted host cell, and to express the transduced gene at significant levels. Therefore, vectors derived from the gamma-retroviruses including, for example, the murine leukemia virus (MLV), have become a standard tool in gene transfer technology and have been frequently used in clinical gene therapy trials (ROSS, G, et al. Gene therapy in the United States: a five-year status report. Hum Gene Ther. 1996, vol.7, no.14, p.1781-90.). Moreover, the use of these vectors for the treatment of different monogenetic immunodeficiencies has also been previously described.

Pseudotyping of retroviral vectors, including HIV vectors or MLV vectors, refers to the incorporation of envelope proteins from heterologous viruses into the retroviral envelope membrane. Such pseudotyped retroviral vectors then exhibit a receptor phenotype similar to the virus from which the envelope protein was derived. Depending on the host range of said virus, the pseudotyped retroviral vectors offer a broadened or a narrowed host range as compared to vector particles having the incorporated homologous retroviral envelope proteins.

A number of pseudotyped vectors are known, including pseudotyped MLV vectors containing a HIV Env protein, an Ebola virus glycoprotein, or a baculovirus glycoprotein.

While vector particles derived from C-type retroviruses can only transduce genes into mitotically active cells, lentiviral vectors are capable of transducing actively proliferating cells as well as non-proliferating cells. However, most of the known lentivirus-derived vector particles are only capable of transducing cells residing in the G1 phase of the cell cycle, therefore such vector particles have been unavailable for transducing cells existing in the G0 phase including, for example, various stem cells, which would otherwise be therapeutically useful target cells in, *interalia,* gene therapy paradigms.

Accordingly, one desirable application of these lentiviral vectors is the stable transduction of cells, for example monocytes, residing in the G0 phase of the cell cycle that has not been possible before. Currently, and as an example, the pulsing of dendritic cells with tumor antigens or viral material must be carried out in an extremely advanced state of cell differentiation in known vaccination approaches (LU, W, et al. Therapeutic dendritic-cell vaccine for chronic HIV-1 infection. Nat Med. 2004, vol. 10, no.12, p.1359-65.) As a further example, both culturing which involves the activation or differentiation of quiescent cells) in addition to a subsequent transduction at a substantial MOl value is currently necessary in order to achieve an acceptable transduction efficiency. Such requirements entail various disadvantages as culturing and activation may lead to undesirable changes in the physiological properties of the cells, including possible alterations in the specific properties and functions the cells, such as stem cells. The culturing and activation of, for example, lymphocytes is often accompanied by the loss of specific functions or the loss of expression of specific surface receptors, for example, the loss of CD4 helper cell functionality due to a downregulation of the CD4 receptor. Additionally, transduction at a high MOl value typically leads to the unwanted risk of insertional mutagenesis (WOODS, NB, et al. Lentiviral vector transduction of NOD/SCID repopulating cells result in multiple vector integrations per transduced cell: risk of insertional mutagenesis. Blood. 2003, vol.101, no.4, p.1284-9.).

Therefore, especially in view of the great importance of pseudotyped retroviral vectors in numerous gene transfer technology and clinical applications, there is still a significant need for additional improved and safe pseudotyped retroviral vectors capable of transducing quiescent cells in the G0 phase of the cell cycle and methods for generating and using such vectors.

### Disclosure of Invention

The vector particles of the present invention that are based on the H IV-2 virus and pseudotyped with the VSV-G protein provide a means of effectively transducing cells residing in the G0 phase of the cell cycle. Unexpectedly, it has been found that said vector particles of the present invention permit the efficient transduction of cells residing in the G0 phase of the cell cycle at a low MOl value thus avoiding the risk of insertional mutagenesis. Particularly, the MOl value used for transduction can be as low as 2. Such an unexpectedly low MOl value is not possible with any other known retrovirus derived vector particle used for the transduction of cells residing in the G0 phase. More particularly, the vector particles of the present invention provide a means for the safe transduction of completely quiescent cells, including stem cells or precursor cells or preferably monocytes, T cells, B cells, CD34+ cells, CD34+/CD38+ cells, neural stem cells or so called "cancer stem cells" which may reside in a tumor cell population (GROSZER, M, et al. PTEN negatively regulates neural stem cell self-renewal by modulating G0-G1 cell entry. Proc. Natl. Acad. Sci. U.S.A.. 2006, vol. 103, no.1, p.111-6.) with a high transduction efficiency and without the additional need to activate said cells by, for example, cell culture media and/or cytokines or growth factors.

The vector particles of the present invention have been shown to promote a more efficient transduction of stem cells residing in the G0 phase of the cell cycle than known vector particles that are based on the highly infectious smmPBj strain of the related lentiviral Simian immunodeficiency virus (SIV; FULTZ, PN, et al. Identification and biologic characterization of an acutely lethal variant of simian immunodeficiency virus from sooty mangabeys (SIV/SMM). AIDS Res Hum Retroviruses. 1989, vol.5, no.4, p.397-409.) that were likewise pseudotyped with the VSV-G protein. Thus, the vector particles of the present invention provide an improved means for the use of such particles in applications including, *interalia,* gene therapy, directed immunotherapy or vaccination paradigms.

Another major advantage of the present vector particles over known vector particles based on, for example, SIV is the fact that HIV-2 is a human pathogen and that thus during treatment with pharmaceutical compositions based on the presently disclosed vector particles no unwanted foreign pathogen is transferred to humans. A further advantage of the HIV-2 virus relating to its safe therapeutic use is that HIV-2 is only able to encapsidate its own RNA while, for example, HIV-1 efficiently encapsidates both HIV-1 and HIV-2 vector constructs. To emphasize, the encapsidation process of the HIV-2 virus is highly specific.

Accordingly, one aspect of the present invention relates to a pseudotyped lentiviral vector particle for the transduction of quiescent cells residing in the G0 phase of the cell cycle, characterized in that said vector particle comprises the Gag, Pol and Vpx proteins of HIV-2 or homologs thereof, the G protein of VSV or homologs thereof and an psi-positive RNA molecule that is derived from HIV-2. In a preferred embodiment, the vector particle further comprises the HIV-2 Vpr, Vif and Nef proteins or homologs thereof. In a further preferred embodiment, the selected cells are animal or human cells and even more preferably are selected from the group consisting of stem cells, neural stem cells, precursor cells, monocytes, T cells, B cells, CD34+ cells, CD113+ cells and/or CD38+ cells. In a further and even more preferred embodiment, the psi-positive RNA molecule is a psi-positive RNA expression vector that comprises at least one gene selected from the group consisting of a selectable marker gene, a gene encoding a viral or bacterial antigen, a gene encoding a tumoral antigen, an antigen frame of an infectious agent and a functional copy of a defective or mutated gene in a patient suffering from an inherited disease.

In a further aspect the present invention relates to a pharmaceutical composition comprising the pseudotyped lentiviral vector particle of the present invention.

Another aspect of the present invention is the use of the pseudotyped lentiviral vector of the present invention for the preparation of a medicament, preferably a vaccine.

In a further aspect, the present invention is directed to the use of the pseudotyped lentiviral vector of the present invention for the preparation of a medicament for gene therapy, specific immunotherapy, vaccination or for the treatment or prevention of at least one condition in a subject, wherein the condition is selected from the group consisting of an inherited monogenetic disease, a cardiovascular disease, a neurodegenerative disease, cancer, HIV, Alzheimer disease, Parkinson disease, diabetis, a neuroinflammatory disease, a rheumatic disease, and an autoimmune disease.

Another aspect of the present invention is a method for producing a pseudotyped lentiviral vector particle, the method comprising: providing a packaging cell line expressing the Gag, Pol and Vpx proteins of HIV-2 or homologs thereof, and the G protein of VSV; transfecting said packaging cell line with a psi-positive HIV-2 transfer vector; and allowing the assembly of pseudotyped lentiviral vector particles. In a preferred embodiment, the packaging cell line further expresses additional accessory HIV-2 proteins, preferably the HIV-2 Vpr, Vif and Nef proteins or homologs thereof. In a further preferred embodiment, the psi-positive HIV-2 transfer vector comprises at least one gene selected from the group consisting of a selectable marker gene, a gene encoding a fluorescent protein, a gene encoding a viral or bacterial antigen, a gene encoding a tumoral antigen, GFP, eGFP, and a functional copy of a defective or mutated gene in a patient suffering from an inherited disease.

A further aspect of the invention is a method of transducing a cell residing in the G0 phase of the cell cycle, the method comprising: providing a pseudotyped lentiviral vector particle according to the present invention; and contacting said pseudotyped lentiviral vector particle with a cell residing in the G0 phase of the cell cycle. In a preferred embodiment, the cell is an animal or human cell and, more preferably, is selected from the group consisting of stem cells, neural stem cells, precursor cells, monocytes, T cells, B cells, CD34+ cells, CD113+ cells and/or CD38+ cells. In an even more preferred embodiment, the cell is contacted with the pseudotyped lentiviral vector particle at an MOl of 2.

In another aspect the present invention is directed to a method for the identification of genes expressed specifically during the G0 phase of the cell cycle, the method comprising the steps of: (a) providing a population of cells; (b) dividing the population of cells of step (a) into two subpopulations; (c) transducing one subpopulation of cells of step (b) with an HIV-1 based vector particle carrying a selectable marker gene and transducing the other subpopulation of cells of step (b) with an HIV-2-based vector particle according to the present invention, wherein said HIV-2-based vector particle is further carrying a selectable marker gene;
(d) isolating cells of both subpopulations of step (c) that express the transduced selectable marker gene; (e) determining the transcriptomes of the cells selected in step (d); and (f) identifying those genes preferably expressed by the transduced cells of the second subpopulation, wherein said genes preferably expressed by the transduced cells of the second subpopulation are specifically expressed during the G0 phase of the cell cycle. In a preferred embodiment the method is directed to the identification of genes coding for cell surface proteins specifically expressed during the G0 phase of the cell cycle. In a further preferred embodiment the method is directed to the generation of antibodies directed against a protein expressed specifically in the G0 phase of the cell cycle, wherein the method further comprises: (g) expressing at least one of the genes identified in step (f) to produce a recombinant protein; and (h) using the recombinant protein to generate antibodies directed against said protein.

Additional embodiments of the invention are recited in the appended claims.

### Brief Description of Drawings

**FIGURE 1** is a schematic overview of the PBj-derived two-plasmid system. Figure 1A depicts the PBjΔE-EGFP construct; Figure 1 B depicts the pMD.G construct.

**FIGURE 2** is a schematic overview of the PBj-derived three-plasmid system. Figure 2A depicts the PBj-derived transfer vector (PBj trans); Figure 2B depicts the packaging construct (PBj pack); Figure 2C depicts the expression construct used to supply the VSV-G protein (pMD.G); and Figure 2D depicts the Vpx expression construct supplying the Vpx protein. In other embodiments, vector particles harbouring Vpx are generated by co-transfection using an expression plasmid coding for Vpx.

**FIGURE 3** is a schematic overview of the HIV-2-derived three-plasmid system. Figure 3A depicts the HIV-2-derived transfer vector (HIV-2 trans); Figure 3B depicts the packaging construct (HIV-2 pack); the third vector is the expression construct pMD.G as shown in Fig. 2C.

**FIGURE 4** shows the results of a fluorescence microscopy analysis of primary alpha-1 cells transduced with HIV-2-derived VSV-G pseudotyped vector particles, PBj-derived VSV-G pseudotyped vector particles and HIV-1-derived VSV-G pseudotyped vector particles.

**FIGURE 5** shows the results of a fluorescence microscopy analysis of PBMCs transduced with HIV-2-derived VSV-G pseudotyped vector particles (▲), PBj-derived VSV-G pseudotyped vector particles (■) and HIV-1-derived VSV-G pseudotyped vector particles (◆).

### Detailed Description of the Invention

### Definitions

The term "**vector particles**", as used in the present invention, refers to replication deficient vectors. Thus, the lentiviral vector particles of the present invention are lentiviral vectors that contain an RNA which, upon the infection of a host or target cell, is reverse-transcribed and inserted into the genome of said cell. However, these vector particles only contain an incomplete genome of the lentivirus from which they are derived. Typically, the RNA molecule of the vector particle does not itself comprise the complete genetic information derived from the *gag, env,* or *pol* genes, which is a known minimal requirement for successful replication of a retrovirus. As a result, the vector particles are replication deficient, meaning that they are not able to transduce a host or target cell with the genetic information required for their own replication.

A vector particle thus comprises a minimum of the Gag, Pol, and Env proteins and an RNA molecule which can be an expression vector. The Gag, Pol, and Env proteins required for assembling the vector particle are derived from the retrovirus and are provided *in trans* by means of a packaging cell line such as, for example HEK-293T. The RNA molecule is derived from the genome of the retrovirus but does not comprise at least one of the *gag, env,* or *pol* genes itself. However, to produce a safe and efficient vector particle, in general all three of said genes are absent, as well as some or all of the other nonessential genes. With respect to HIV-2, such nonessential genes comprising the HIV-2 genome that can be deleted from the RNA molecule include *rev,tat, vif, vpr, vpx* and *nef.*

However, the RNA molecule still comprises all cis-acting elements, for example the psi element and LTRs, derived from the retroviral genome that are required for an effective processing and packaging of the RNA into the resulting vector particles. For an expression vector, the RNA molecule preferably comprises a further gene, usually heterologous gene, that is under the control of a suitable promoter, for example, the CMV promoter, and is thus expressed upon integration of the gene into the genome of the host or target cell.

Accordingly, one example of an RNA molecule or transfer vector that may be suitable to generate a retroviral vector particle is based on the HIV-2 genome and comprises the LTRs, the psi element and the CMV promoter followed by the gene to be transduced, for example, the gene for a GFP or eGFP protein. The sequence of the HIV-2 virus has been published by KIRCHHOFF, F, et al. A novel proviral clone of HIV-2: biological and phylogenetic relationship to other primate immunodeficiency viruses. Virology. 1990, vol.177, no.1, p.305-11. The *gag, env* and po/genes of HIV-2 are removed or expression of their gene products is prevented by events including a frame shift mutation. The minimal requirements for a lentivirus vector based on HIV-1 have been described by Kim *et al.* (KIM, VN, et al. Minimal Requirement for a Lentivirus Vector Based on Human Immunodeficiency Virus Type 1. J Virol. 1998, vol.72, p.811-16.).

The above-described RNA molecule together with the HIV-2 *gag, pol* and *env* proteins, provided *in trans* by the packaging cell line, are then assembled into the vector particles or vector particles, which will be able to efficiently infect their target or host cells, reverse-transcribe the RNA molecule that may comprise a heterologous gene under the control of the CMV promoter, and integrate said genetic information into the genome of the target/host cells. However, as the genetic information for the *gag, pol* and *env* proteins is not present on the transduced RNA molecule, the vector particles or vector particles will be replication deficient, meaning that no new generation of said vector particles will thus be produced by the transduced cell.

The terms **"pseudotyped", "pseudotyped vector"** or **"pseudotyped vector particle**", as used in the present invention, refer to a vector particle bearing an envelope glycoprotein derived from other viruses having an envelope. The host range of the lentiviral vectors vector particles of the present invention can thus be expanded or altered depending on the type of cell surface receptor used by the glycoprotein.

As previously described *supra,* the *gag, po*/ and *env* proteins needed to assemble the vector particle are provided *in trans* by means of a packaging cell line, for example, a HEK-293T cell line. This is typically accomplished by transfecting the packaging cell line with one or more plasmids containing the *gag, pol* and *env* genes. For the generation of pseudotyped vectors, the *env* gene, originally derived from the same retrovirus as the *gag* and *pol* genes and as the RNA molecule or expression vector, is exchanged for the envelope protein(s) of a different enveloped virus, for example, the spike glycoprotein of the vesicular stomatitis virus (VSV-G; BURNS, JC, et al. Vesicular stomatitis virus G glycoprotein pseudotyped retroviral vectors: concentration to very high titer and efficient gene transfer into mammalian and nonmammalian cells. Proc. Natl. Acad Sci. U.S.A.. 1993, vol.90, no.17, p.1833-7.) may be used.

While retroviral infection typically requires the interaction between the viral envelope protein and specific cell surface receptor proteins, VSV-G interacts with a phospholipid component of the cell membrane to mediate viral entry by membrane fusion (MASTROMARINO, P, et al. Characterization of membrane components of the erythrocyte involved in vesicular stomatitis virus attachment and fusion at acidic pH. J Gen Virol. 1998, vol.68, no.9, p.2359-69.) and viral entry seems not to be dependent on the presence of specific protein receptors. Consequently, VSV has an extremely broad host-cell range (MARSH, M, et al. Virus entry into animal cells. Adv Virus Res. 1989, vol.107, no.36, p.107-51.).

Thus, an exemplary pseudotyped vector particle based on the HIV-2 retrovirus comprises the (1) HIV-2 Gag, Pol and vpx proteins, (2) an RNA molecule derived from the HIV-2 genome that may be used to generate a retroviral vector particle based on the HIV-2 genome lacking the *gag, env, pol, tat, vif, vpr, vpx* and *nef* genes, but still comprising the LTRs, the psi element and a CMV promoter followed by the gene to be transduced, for example, a gene coding for the GFP protein, and (3) the G protein of VSV.

The resulting pseudotype vector particle will thus display the typical tropism characteristics of VSV thus imparting to the vector particle a broad host-cell range as the G-protein is thought to interact with a ubiquitous cellular receptor that is known to be an abundant membrane phospholipid.

A pseudotyped vector particle **derived from**, for example, HIV-2, as used in the present invention, refers to a vector particle in which the genetic information for the RNA and/or the Gag and Pol proteins comprising the vector particle originates from said retrovirus, for example, the HIV-2 retrovirus. As described above, the original retroviral genome can comprise mutations, such as deletions, frame shift mutations and insertion sequences.

The person skilled in the art will readily be able to introduce mutations as, for example, additions and deletions, into a given nucleic acid or amino acid sequence. Such known methodologies are, for example, disclosed in Sambrook *et al.* (SAMBROOK, J, et al. Molecular Cloning. A Laboratory Manual. 2nd edition. Edited by SAMBROOK, J, et al. Cold Spring Harbor: Cold Spring Harbor Laboratory, 1989.).

The invention preferably includes nucleic acids sequences or homologs thereof, or unique fragments thereof. In the present invention, the sequence of a nucleic acid molecule encoding a resulting protein is considered to be homologous to a second nucleic acid molecule if the nucleotide sequence of the first nucleic acid molecule is at least about 70% homologous, preferably at least about 80% homologous, and more preferably at least about 85%, 90%, 95% or 99% homologous to the sequence of the second nucleic acid molecule. Homology between two nucleic acid sequences may be readily determined using the known BLASTN algorithm with default settings (ALTSCHUL, SF, et al. Basic local alignment search tool. J Mol Biol. 1990, vol.215, no.3, p.403-10.). As a further example, another known test for ascertaining the homology of two nucleic acid sequences is whether they hybridize under normal hybridization conditions, preferably under stringent hybridization conditions.

The protein encoded by the nucleic acid of the present invention further includes functional homologs. A protein is considered a functional homolog of another protein for a particular function, if the homolog has a similar physiological function as the original protein. The homolog can be, for example, a fragment of the protein, or a substitution, addition, or deletion mutant of the protein.

Determining whether two amino acid sequences are substantially homologous is typically based on a FASTA search in accordance with Pearson & Lipman (PEARSON, WR , et al. Improved tools for biological sequence comparison. Proc Natl Acad Sci U S A. 1988, vol.85, no.8, p.2444-8.). For example, the amino acid sequence of a first protein is considered to be homologous to that of a second protein if the amino acid sequence of the first protein shares at least about 70% amino acid sequence identity, preferably at least about 80% identity, and more preferably at least about 85%, 90%, 95% or 99% identity, with the sequence of the second protein.

Using the nucleic acid sequences disclosed herein, the skilled person can further design nucleic acid structures having a particularly desired function suitable in various types of biological applications. For example, oligonucleotides or polynucleotides for use as primers in nucleic acid amplification procedures can be constructed via the known polymerase chain reaction (PCR), ligase chain reaction (LCR), Repair Chain Reaction (RCR), PCR oligonucleotide ligation assay (PCR-OLA), and the like. Oligonucleotides useful as probes in hybridization studies, such as *in situ* hybridization, can also be constructed accordingly. Numerous methods for labeling the probes with radioisotopes, fluorescent tags, enzymes, and binding moieties (e.g., biotin) are known, thus the probes of the present invention can be adapted for detectability in a straightforward manner.

The terms "**Psi positive**" and "**psi negative**", as used in the present disclosure, refer to a nucleic acid molecule wherein the retroviral psi element is present and absent, respectively. The psi element is a known cis-acting signal located near the 5' end of the retroviral genome and designates a packaging signal, which is of importance during assembly of the viruses and leads to the incorporation of the viral RNA into the viral core. Thus, a psi negative RNA does not comprise the retroviral psi element and consequently will not be assembled into a vector particle of the present invention. By contrast, a psi positive RNA that does comprise said psi element will be effectively assembled into the vector particle.

The terms "**Titers**" and "**transduction efficiency**" refer to the ability of a vector particle to enter a cell and integrate the genetic information, in particular RNA information, into the genome of the cell. Thus, the terms titer and transduction efficiency can be used synonymously. The titer/transduction efficiency can, for example, be determined by incorporation of a gene coding for a detectable marker, for example, under the control of a CMV promoter, into the expression vector of the vector particle. Upon transduction of such a detectable marker by means of the vector particle, the transduced cells will then express the detectable marker. If the detectable marker is, for example, the GFP protein, the number of cells infected by a given number of vector particles can readily be determined by techniques, including FACS analysis, that directly corresponds to the titer or transduction efficiency of the vector particle that has been used for transduction. The titer or transduction efficiency of a given vector particle thus refers to the number of cells transduced relative to the number of vector particles used.

The titer or transduction efficiency is used as a means to characterize and compare vector particles with regard to their ability to transduce a target cell. Thus, a vector particle having an increased titer or an increased transduction efficiency is able to transduce a higher number of cells at a given vector particle concentration than a different vector particle having the same vector particle concentration.

The terms "**quiescent**" and "**G0 phase**" as used in the present disclosure refer to specific phases of the cell cycle. The cell cycle is divided into two main parts: interphase and mitosis. During interphase, the cell grows and replicates its chromosomes. Interphase accounts for all but an hour or two of a typical 24-hour cell cycle, and is subdivided into three phases: gap phase 1 (G1), synthesis (S), and gap phase 2 (G2). Interphase is followed by mitosis (known as nuclear division) and cytokinesis (commonly referred to as cell division). This relatively brief part of the cell cycle includes some of the most dramatic events in cell biology. The G1 begins at the completion of mitosis and cytokinesis and lasts until the beginning of S phase. This phase is generally the longest of the four cell cycle phases and is quite variable in length. During this phase, the cell chooses either to replicate its deoxyribonucleic acid or to exit the cell cycle and enter a quiescent state known as the G0 phase. Nonproliferative cells in multicellular eukaryotes generally enter the quiescent G0 state from G1 and may remain quiescent for long periods of time, possibly indefinitely, a common scenario for cells that are fully differentiated. However, also undifferentiated cells such as, *interalia,* stem cells, progenitor cells or monocytes can reside in the G0 phase of the cell cycle. If such cells are subjected to the right stimulus, for example because they have migrated into a specific part of the body and get in contact with specific cytokines or cells, they may leave the G0 phase to further differentiate or proliferate. During the G0 phase, the cell cycle machinery is dismantled and cyclins and cyclin-dependent kinases disappear. Moreover, quiescent cells reveal low nucleotide levels which accounts for the obstacles a retrovirus has to overcome in order to integrate its genomic information into the genome of a target cell residing in the G0 phase.

The terms "**plasmid**", "**construct**", "**vector**", "**expression vector**", "**packaging vector**", and "**transfer vector**" as used in the present disclosure refer to DNA molecules used to produce the vector particles of the present invention. Typically, the terms "plasmid", "construct" and "vector" are used synonymously. Specifically, an expression vector is a construct that contains at least one gene to be expressed under the control of at least one promoter. If an expression vector is transfected into a suitable cell, for example, a HEK-293T cell, the genomic information of the gene to be expressed is transcribed into RNA that is consequently translated into the corresponding gene product by the translational machinery of the transfected cell. A packaging vector likewise is an expression vector that harbours one or more genes for the viral proteins needed to assemble a virus particle, for example, the *gag*/*pol* gene. A transfer vector is derived from a viral genome, preferable a retroviral genome and more preferable the HIV-2 genome and provides the RNA to be assembled into the generated vector particles when co-transfected into a suitable cell, for example, a HEK-293T cell. Typically, a transfer vector is generated from the complete genome of said retrovirus wherein various genetic elements have been deleted or mutated to prevent their expression. However, and as explained in greater detail below, a transfer vector comprises all the necessary cis-active elements to ensure an effective packaging of the transcribed RNA into the lentiviral vector particles to be produced (*inter alia* the psi element and LTRs). Moreover, the transfer vector typically comprises a heterologous gene under the control of a promoter which is to be integrated into the genome of the target cell and to be consequently expressed by said target cell.

The terms "**cell marker**", or "**cell surface marker**", as used in the present invention, refer to a molecule present on the surface of a cell. Such molecules can be, *interalia,* peptides or proteins that may comprise sugar chains or lipids, antigens, clusters of differentiation (CDs), antigens, antibodies or receptors. Since not all populations of cells express the same cellular markers, a cell marker can thus be used to identify, select or isolate a given population of cells expressing a specific cell marker. As an example, CD34 is a cell marker typically expressed by peripheral blood progenitor cells (PBPCs). Thus, PBPCs can be effectively identified, selected or otherwise isolated, *inter alia* by a FACS cell sorter, by means of the CD34 cell marker. Further examples for cell markers are CD38 and CD133, or CD14 found on monocytes.

The terms "**selectable marker gene**" or "**selectable marker**", as used in the present invention, refer to a gene and its corresponding product that permit detection, selection and/or isolation of a desired product. If such a selectable marker is expressed by a cell, consequently such a cell or a population of such cells can be detected, selected and/or isolated.

Selectable markers can include, *interalia,* a molecule, preferably a peptide or protein, detectable by fluorescence, an enzyme catalyzing a reaction where the resulting product is monitored, a molecule that confers a resistance to, *interalia,* an antibiotic or another physiologically toxic substance, or a molecule interacting with a non-toxic substance to thereby render the substance toxic. Detection, selection and/or isolation can be carried out, *interalia,* using FACS, a FACS cell sorter, use of fluorescence microscopy, or by the use of antibiotics or cytotoxic substances, or using precursors of cytotoxic substances.

### Taxonomy

Viruses of the taxonomic family of the *Retroviridae* share, as a unifying feature, a positive stranded ssRNA genome (herein "ss(+)RNA"). During infection, said ss(+)RNA can not directly be used as a template (mRNA), thus the genetic information of this virus is first reverse-transcribed from RNA into DNA by a reverse transcriptase provided by the virus and subsequently integrated into the host genome. Thus, the term "retro" refers to the activity of reverse transcriptase and the transfer of genetic information from RNA to DNA. Presently, more than 570 individual viruses of the family have been identified.

One sub-family of *Retroviridae* comprises the *Orthoretroviridae* viruses, including the genera of the alpha-retroviruses with, for example, the Avian leukosis virus (ALV) species the gamma-retroviruses with, for example, the Murine leukemia virus (MLV) species or the Feline leukemia virus (FLV) or the delta-retroviruses with, for example, the Bovine leukemia virus (BLV) species or the Human T-lymphotropic virus (HTLV).

One additional genus of the *Orthoretroviridae,* known as the lentiviruses, has recently attracted much interest largely because of a well-known member, the Human immunodeficiency virus (HIV). However, the genus of lentiviruses comprises numerous other viruses. In addition to the HIV-1 or HIV-2 species, the Bovine immunodeficiency virus (BIV), the Feline immunodeficiency virus (FIV) and the Simian immunodeficiency virus (SIV) are all examples of frequent and extensive research efforts. A general overview of the retroviruses and lentiviruses, respectively can be found, for example, in Vigna *et al.* and Palu *et al.* (VIGNA, E, et al. Lentiviral vectors: excellent tools for experimental gene transfer and promising candidates for gene therapy. J Gene Med. 2000, vol.2, no.5, p.308-16. , PALU, G, et al. Progress with retroviral gene vectors. Rev Med Virol 2000, vol.10, no.3, p.185-202.)

### Structural and Genetic Features of Lentiviral Genomes

The genomes of known replication-competent retroviruses typically comprise the four coding domains (*gag, pro, pol, env*):

The first of the four coding domains (*gag*) encodes a polypeptide (herein "Gag") wherein the cleavage products comprise the major structural proteins of the virus core including Matrix (MA), which is associated with the inside of the virus envelope; capsid (CA), a principal structural protein of the virion core; and nucleocapsid (NC), a small, basic protein which is tightly bound to the viral genomic RNA and forming a ribonucleoprotein complex within the core.

The second of the four coding domains (*pro*) encodes part of the polyprotein (Gag-Pro or Gag-Pro-Pol) wherein the cleavage products typically include protease (PR) and occasionally dUTPase (DU).

The third of the four coding domains (*pol*) encodes part of the polyprotein (Gag-Pro-Pol), wherein the cleavage products typically include reverse transcriptase (RT) and integrase (IN) and, in some lentiviruses, dUTPase (DU). In spumaviruses, *po*/ is expressed via a spliced mRNA as Pol-Pol polyprotein.

Finally, e*nv* encodes a polyprotein (Env) whose cleavage products SU (surface) and TM (transmembrane) comprise the structural proteins of the viral envelope. These proteins are usually not required for the assembly of enveloped viral vector particles, but they do have an essential role in the cellular entry process of such particles. The SU domain binds to a specific receptor molecule on the target cell. This binding event appears to activate the membrane fusion-inducing potential of the TM protein and, by a process that remains largely undefined, the viral and cell membranes then fuse. The specificity of the SU-receptor interaction defines the host range and tropism of a retrovirus. As a consequence, viral vector particles lacking envelope glycoproteins are noninfectious, and cells lacking a receptor are nonpermissive for viral entry. Viruses may bind weakly to resistant cells through relatively nonspecific interactions, but, in the absence of a specific receptor molecule, they are unable to initiate the infection process.

Furthermore, retroviruses typically contain two long terminal repeats (LTRs) and a region of several hundred (~300-1800) base pairs composed of U3-R-U5 (5to 3) which are located at both ends of the unintegrated and integrated (proviral) genome.

Additionally, a psi element is generally present within the retroviral genome. The psi element is a cis-acting signal, located near the 5' end of the genome and designates a packaging signal, which is of importance during virus assembly and leads to the incorporation of the viral RNA into the viral core.

More complex retroviruses typically comprise additional regulatory genes. In case of HIV-2, these regulatory genes include *ref, tat, vif, nef, vpr* and *vpx.*

### Lentiviral Vector Particles and their Generation

Lentiviral vector particles are replication deficient lentiviral vectors and are useful for transducing a nucleic acid sequence into a target/host genome. Such vector particles comprise a minimum of the Gag, Pol and Env proteins and an RNA molecule, which can be an expression vector. This RNA molecule or expression vector is usually derived from the genome of the original retrovirus, meaning that it comprises all the necessary cis-active elements for effective packaging into the resulting lentiviral vector particles (*inter alia* the psi element and LTRs). However, in order for the lentiviral vector particle to be replication deficient, the RNA molecule does not itself comprise the genetic information of the *gag, env,* or *pol* genes. Instead, a normally heterologous nucleic acid sequence to be integrated into the target/host genome is present in the expression vector. The minimal requirements for a lentivirus vector based on HIV-1 have been described by, for example, Kim et al. (*supra*).

Thus, for generating lentiviral vector particles, three basic components, usually provided on separate plasmids, are required: an expression vector comprising the psi-negative sequence of the *gag*/*pol* gene (also known as a packaging vector), an expression vector comprising the psi-negative sequence of the *env* gene and a psi-positive vector derived from the lentiviral genome (also known as a transfer vector if the vector comprises a gene to be transduced). Co-transfection of these components into a packaging cell line, i.e. a suitable eukaryotic cell line, will lead to the expression of the Gag, Pol and Env proteins as well as generation of psi-positive RNA. However, use of cell lines that are stably transfected with one or more of these basic components is herein contemplated. Since the *gag*/*pol* and *env* genes are psi-negative, the mRNA generated prior to expression of the corresponding proteins will not be assembled into the lentiviral vector particles. By contrast, the psi-positive RNA transcribed from the expression vector will be included in the resulting lentiviral vector particle.

Consequently, replication deficient lentiviral vector particles will be generated comprising the Gag, Pol and Env proteins provided *in trans* by the packaging cell line, as well as the psi-positive RNA transcript of the expression plasmid lacking the genetic information for autonomous replication. However, as the lentiviral vector particles comprise the Gag, Pol and Env proteins, such vector particles are capable of efficiently infecting their target/host cells, reverse-transcribing their RNA, and integrating said genetic information into the genome of the target/host.

### Pseudotyped Heterologous Lentiviral Vector Particles

The host range of lentiviral vectors can be expanded or altered by a process known as pseudotyping. The process of pseudotyping a lentivirus or a lentiviral vector particle is generally understood to result in a lentiviral vector particle that consists of a lentiviral vector particle bearing glycoproteins derived from other enveloped viruses. Such vector particles possess the tropism characteristics of the virus from which the glycoprotein was derived.

As described above, for generating lentiviral vector particles, three basic components, usually provided on separate plasmids, are required. Within a packaging cell line, a psi-negative *gag*/*pol* gene of the original lentivirus, a psi-negative *env* gene of the original lentivirus, and a psi-positive expression vector are needed. For pseudotyped lentiviral vector particles, the envgene of the original retrovirus is replaced by one or more envelope gene(s) of another enveloped virus, for example, the gene coding for G protein of VSV. Furthermore, the RNA transcribed from the psi-positive expression vector is assembled into the resulting lentiviral vector particle. This psi-positive expression vector is usually derived from the genome of the original retrovirus, which means that it comprises all the necessary elements for an effective packaging into resulting pseudotyped lentiviral vector particles. Furthermore, this expression vector may carry at least one gene which is to be transduced, i.e. integrated into the genome of the target cell and ultimately expressed by said cell.

Several of the proteins required to assemble pseudotyped lentiviral vector particles are known to be detrimental to mammalian cells when they are overexpressed. Thus, for the stable production of pseudotyped lentiviral vectors particles, 293-based cell lines allowing conditional production of virus-encoded proteins using tetracycline regulated promoters were generated (XU, K, et al. Generation of a stable cell line producing high-titer self-inactivating lentiviral vectors. Mol Ther. 2001, vol.3, no.1, p.97-104.). In one embodiment, the packaging cell used for producing the pseudotyped lentiviral vector particles of the present invention is H EK-293T.

The psi element referred to above designates a packaging signal, which leads to the incorporation (the packaging or encapsidation) of the RNA transcribed from the expression vector into the vector particles during the assembly of the vector particles. Thus, psi designates the retroviral packaging signal that efficiently controls the packaging of the messenger RNA of the expression vector. In order for packaging to occur, the expression vector also has to be flanked by specific long terminal repeat sequences (LTRs), so the transcription of the RNA of the expression vector into DNA and subsequent integration into the genome of the target cell can successfully be accomplished.

Consequently, during assembly of the pseudotyped lentiviral vector particles, neither the RNA of the *gag*/*pol* gene nor that of the envelope gene encoding, for example, the VSV-G protein is incorporated into the lentiviral vector particles. These genes are only transcribed into RNA by the transcription machinery of the packaging cell and further translated into functional proteins. Contrasting this, the psi-positive expression vector is translated into the corresponding RNA and, due to the presence of the psi element and the LTRs, is incorporated into the resulting lentiviral vector particles and ultimately transduced into the genome of the host cell.

### The Lentiviral Vector Particles of the Present Invention

The lentiviral vector particles of the present invention are typically produced based on psi-positive lentiviral expression vectors, preferably an HIV-2 expression vector, and comprising the Vpx protein of HIV-2.

Unexpectedly, it was found that such lentiviral vector particles pseudotyped with the VSV-G protein enable a highly efficient gene transfer into G0 quiescent cells such as, *inter alia,* stem cells, neural stem cells, precursor cells, monocytes, T cells, B cells, CD34+ cells, CD113+ cells and/or CD38+ cells using a low MOl, which entails the advantage of a very low risk of insertion mutagenesis and adds to the safety of such pseudotyped lentiviral vector particles. The transduction efficiency of the pseudotyped HIV-2 based particles of the present invention into quiescent cells in the G0 phase of the cell cycle was found to be considerably higher than of vector particles based on HIV-1 or SIV-PBj pseudotyped with the VSV-G protein.

Even though any lentiviral expression vector may suitably be used according to the present invention, the lentiviral expression vector used for the production of lentiviral vector particles is preferably based on HIV-2. It will be obvious for the skilled person that the lentiviral expression vectors need not comprise the complete genomic information of the respective lentivirus; rather these lentiviral vectors only need to comprise the necessary elements for effective packaging into the resulting lentiviral vector particles as described above.

In one embodiment of the invention the pseudotyped lentiviral vector particles comprise the HIV-2 Gag, Pol, Vpr, Vif and Nef proteins, the VSV-G protein and a psi-positive RNA molecule that is derived from the HIV-2 genome and comprises the psi element, a heterologous gene under control of a promoter and flanking LTRs. In a preferred embodiment, said RNA molecule further comprises the *vpr, vif, nef* and *vpx* genes and a mutation in the *gag*/*pol* gene preventing its expression. In an even more preferred embodiment, the heterologous gene is coding for GFP or eGFP and/or is under the control of the CMV promoter.

In order to produce the present vector particles, the accessory proteins Vpx, Vpr, Vif and Nef can be provided to the packaging cell in different ways. They can independently be present on the transfer vector, on the packaging vector and/or on a separate expression plasmid. Thus, in an exemplary further embodiment, the *vpx* gene is present on a separate expression vector, while the *vpr, vif* and *nef* genes are encoded by the transfer vector. Moreover, some or all of the accessory proteins can be stably expressed by the packaging cell.

As demonstrated in the appended Examples, the vector particles of the present invention exhibit a significantly increased and improved transduction efficiency for cells, for example monocytes, CD34+ cells or CD34+/CD38+ cells, residing in the G0 phase of the cell cycle as compared to vector particles based on, for example, SIV or HIV-1. Moreover, the present vector particles are capable of efficiently transducing target cells residing in the G0 phase of the cell cycle at an MOl as low as 2. Thus, the pseudotyped vector particles of the present invention can be used at a very low yet safe MOl value in a variety of clinical applications.

### Pharmaceutical Compositions and Medical Uses Based on the Lentiviral vector particles of the Present Invention

### Formulations and Routes of Administration

In a further embodiment, a pharmaceutical composition based on the present lentiviral vector particles can be formulated in any conventional manner using one or more physiologically acceptable carriers or excipients. Thus, in a further embodiment of the invention, the lentiviral vector particles of the present invention may be formulated for administration by, for example, injection, inhalation or insulation, for example, orally, or intra-nasally or by buccal, parenteral or rectal administration.

In a further embodiment, the pharmaceutical compositions of the present invention can be formulated for a variety of modes of administration, including systemic, topical or localized administration. Techniques and formulations can be found in, for example, GENNARO, AR. Remington's Pharmaceutical Sciences. 18th edition. Edited by GENNARO, AR. Easton, PA: Mack Pub. Co., 1995. For systemic administration, injection is preferred, including intramuscular, intravenous, intraperitoneal, and subcutaneous. For the purposes of injection, the pharmaceutical compositions of the present invention can be formulated in liquid solutions, preferably in physiologically compatible buffers, such as Hank's solution or Ringer's solution. In addition, the pharmaceutical compositions may be formulated in solid form and redissolved or suspended immediately prior to use. Lyophilized forms of the pharmaceutical composition are also suitable.

In a further embodiment of oral administration, the pharmaceutical compositions of the present invention may take the form of, for example, tablets or capsules prepared by conventional means with pharmaceutically acceptable excipients such as binding agents (e.g. pregelatinized maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycolate); or wetting agents (e.g. sodium lauryl sulfate). The tablets can also be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. ationd oil, oily esters, ethyl alcohol or fractionated vegetable oils); and preservatives (e.g. methyl or propyl-p-hydroxybenzoates or sorbic acid). The preparations can also contain buffer salts, flavoring, coloring and sweetening agents as appropriate.

In a further embodiment, the pharmaceutical compositions can be formulated for parenteral administration by injection, e.g. by bolus injection or continuous infusion. Formulations for injection can be presented in a unit dosage form, e.g. in ampoules or in multi-dose containers, with an optionally added preservative. The pharmaceutical compositions can further be formulated as suspensions, solutions or emulsions in oily or aqueous vehicles, and may contain other agents including suspending, stabilizing and/or dispersing agents. Additionally, the pharmaceutical compositions can also be formulated as a depot preparation. These long acting formulations can be administered by implantation (e.g. subcutaneously or intramuscularly) or by intramuscular injection. Thus, for example, the compounds may be formulated with suitable polymeric or hydrophobic materials (e.g. as an emulsion in an acceptable oil) or ion exchange resins, or as sparingly soluble derivatives, for example, as a sparingly soluble salt. Other suitable delivery systems include microspheres, which offer the possibility of local noninvasive delivery of drugs over an extended period of time. This technology can include microspheres having a precapillary size, which can be injected via a coronary catheter into any selected part of an organ without causing inflammation or ischemia. The administered therapeutic is then slowly released from the microspheres and absorbed by the surrounding cells present in the selected tissue.

Systemic administration can also be accomplished by transmucosal or transdermal means. For transmucosal or transdermal administration, penetrants appropriate to the barrier to be permeated are used in the formulation. Such penetrants are generally known in the art, and include, for example, for transmucosal administration, bile salts, and fusidic acid derivatives. In addition, detergents may be used to facilitate permeation. Transmucosal administration can occur using nasal sprays or suppositories. For topical administration, the vector particles of the invention can be formulated into ointments, salves, gels, or creams as generally known in the art. A wash solution can also be used locally to treat an injury or inflammation in order to accelerate the healing process.

In another embodiment, the invention is directed to a pharmaceutical composition comprising the pseudotyped vector particles of the present invention, or the use of the pseudotyped vector particles of the present invention for the preparation of a medicament. Preferably, the pharmaceutical composition further comprises a pharmaceutically acceptable carrier or excipient.

The pharmaceutical compositions of the present invention can be administered alone or in combination with other types of cancer treatment strategies (e.g., radiation therapy, chemotherapy, hormonal therapy, immunotherapy and anti-tumor agents). Examples of anti-tumor agents include, but are not limited to, cisplatin, ifosfamide, paclitaxel, taxanes, topoisomerase I inhibitors (e. g., CPT-11, topotecan, 9-AC, and GG-211), gemcitabine, vinorelbine, oxaliplatin, 5-fluorouracil (5-FU), leucovorin, vinorelbine, temodal, and taxol.

### Use of the Pseudotyped Vector Particles for Medical Applications

One embodiment of the invention relates to the use of the present pseudotyped vector particles for the preparation of a medicament.

In a further embodiment, the vector particles and pharmaceutical compositions based on said particles of the present invention can be used for gene therapy, i.e. for transducing quiescent target cells in the G0 phase of the cell cycle, which can include, *interalia,* a cancerous cell, a stem cell, progenitor cell or monocytes with a desired gene that, upon integration into the targeted cells' genome, leads to the prevention or the treatment of a particular medical condition. Thus, by gene therapy insertion of genes into cells and tissues to treat a disease, including a hereditary diseases, is possible and a multitude of different methods to replace or repair the genes targeted in gene therapy exist: *interalia,* a normal, i.e. functional and/or non-disease related gene may be inserted into a nonspecific location within the genome to replace a non-functional, abnormal, i.e. disease related gene; the abnormal gene can be exchanged for a normal gene through homologous recombination; the abnormal gene can be repaired through selective reverse mutation, which returns the gene to its normal function; and/or the regulation of a particular gene can be altered. It is important to note that the vector particles of the present invention can be directly used, i.e. they (or a medicament or pharmaceutical composition derived from said particles) can be administered to the subject, or cells, such as stem cells, progenitor cells, monocytes, CD34+ or CD34+/CD38+ cells in the G0 phase, can first be isolated from the subject or a suitable donor, subsequently be contacted with the vector particles of the present invention in order to transduce at least one desired gene and then be introduced into the subject. In other words, the transduction of a cell by the vector particles of the present invention can be carried out *in vivo* or *ex vivo.*

In a further embodiment the pseudotyped vector particles of the invention can be used in specific immunotherapy protocols, wherein therapeutic antigens are transduced into the quiescent cells and, upon expression, result in an increased and/or more specific immune response. Thus if, for example, the therapeutic antigen to be expressed is selected to be a marker gene for a tumor, the induced immune response will consequently be directed against said tumor.

In another embodiment the pseudotyped vector particles of the invention can be used for vaccination, preferably against viral infections. In such a case the gene to be expressed by transduced quiescent cells is a gene of the virus against which immunization is to be achieved. Preferably, the gene encodes for an especially immunogenic protein of said virus. In a preferred embodiment, the gene may encode all or part of the gp120 protein of HIV-1 or all or part of the NS1 protein of influenza virus.

In yet another embodiment, the gene to be transduced into the targeted cells can encode an immune stimulatory protein, and/or be a gene coding for cytostatic, apoptosis-inducing and/or cytotoxic gene products.

In a further embodiment, the gene to be transduced by the pseudotyped vector particle is selected from the group consisting of a selectable marker gene, a gene encoding a fluorescent protein, a gene encoding an antibiotic resistance gene, a gene encoding siRNA, a gene encoding shRNA, a gene encoding an angiogenic factor, a gene encoding an apoptotic factor, a gene encoding a cytotoxic factor, a gene encoding an anti-apoptotic factor, a gene encoding a neuro-protective factor, a gene encoding a viral or bacterial antigen, a gene encoding an anti-viral protein, a gene encoding a tumoral antigen, a gene encoding an immune-stimulatory factor, a functional copy of a defective or mutated gene in a patient suffering from an inherited disease, a gene coding for a cytotoxic protein, including as, *inter alia,* TNF-α, p53an interfering RNA, interferon, an immune stimulatory protein, and/or a gene coding for cytostatic, apoptosis-inducing and/or cytotoxic gene products.

In a further embodiment the invention is directed to the treatment or prevention of cancer, including, but not limited to, neoplasms, tumors, metastases, or any disease or disorder characterized by uncontrolled cell growth, and particularly multidrug resistant forms thereof. Examples of types of cancer and proliferative disorders to be treated with the therapeutics of the invention include, but are not limited to, leukemia (e.g. myeloblastic, promyelocytic, myelomonocytic, monocytic, erythroleukemia, chronic myelocytic (granulocytic) leukemia, and chronic lymphocytic leukemia), lymphoma (e.g. Hodgkin's disease and non-Hodgkin's disease), fibrosarcoma, myxosarcoma, liposarcoma, chondrosarcoma, osteogenic sarcoma, angiosarcoma, endotheliosarcoma, Ewing's tumor, colon carcinoma, pancreatic cancer, breast cancer, ovarian cancer, prostate cancer, squamous cell carcinoma, basal cell carcinoma, adenocarcinoma, renal cell carcinoma, hepatoma, Wilms' tumor, cervical cancer, uterine cancer, testicular tumor, lung carcinoma, small cell lung carcinoma, bladder carcinoma, epithelial carcinoma, glioma, astrocytoma, oligodendroglioma, melanoma, neuroblastoma, retinoblastoma, dysplasia and hyperplasia. In a particular embodiment, therapeutic compounds of the invention are administered to patients having prostate cancer (e.g., prostatitis, benign prostatic hypertrophy, benign prostatic hyperplasia (BPH), prostatic paraganglioma, prostate adenocarcinoma, prostatic intraepithelial neoplasia, prostato-rectal fistulas, and atypical prostatic stromal lesions). The treatment and/or prevention of cancer includes, but is not limited to, alleviating symptoms associated with cancer, the inhibition of the progression of cancer, the promotion of the regression of cancer, and the promotion of the immune response.

While the foregoing medical uses of the vector particles of the present invention have been formulated as methods of treatment of various disorders, it is clear to the skilled person that such wording also comprises the use of the vector particles for the preparation of a medicament for the treatment of such disorders. Therefore, in a further embodiment, the invention is directed to the use of the pseudotyped vector particles of the present invention for the preparation of a medicament for gene therapy, specific immunotherapy, vaccination and/or treatment or prevention of cancer. In another embodiment, the invention is directed to the use of the pseudotyped vector particles of the invention for the preparation of a medicament for the treatment or prevention of at least one condition in a subject, wherein the condition is selected from the group consisting of a chronical infection, an inherited monogenetic disease, a cardiovascular disease, a neurodegenerative disease, cancer, HIV, Alzheimer disease, Parkinson disease, diabetis, a neuroinflammatory disease, a rheumatic disease, an autoimmune disease, adipositas, acute lymphoblastic leukemia, myeloid leukemia, renal carcinoma, and disorders related to endothelialization or re-endothelialization. In yet another embodiment, the cancer is selected from the group consisting of leukemia, myeloblastic, promyelocytic, myelomonocytic, monocytic, erythroleukemia, chronic myelocytic (granulocytic) leukemia, and chronic lymphocytic leukemia, lymphoma, e.g. Hodgkin's disease and non-Hodgkin's disease, fibrosarcoma, myxosarcoma, liposarcoma, chondrosarcoma, osteogenic sarcoma, angiosarcoma, endotheliosarcoma, Ewing's tumor, colon carcinoma, pancreatic cancer, breast cancer, ovarian cancer, prostate cancer, squamous cell carcinoma, basal cell carcinoma, adenocarcinoma, renal cell carcinoma, hepatoma, Wilms' tumor, cervical cancer, uterine cancer, testicular tumor, lung carcinoma, small cell lung carcinoma, bladder carcinoma, epithelial carcinoma, glioma, astrocytoma, oligodendroglioma, melanoma, neuroblastoma, retinoblastoma, dysplasia and hyperplasia, prostate cancer, prostatitis, benign prostatic hypertrophy, benign prostatic hyperplasia (BPH), prostatic paraganglioma, prostate adenocarcinoma, prostatic intraepithelial neoplasia, prostato-rectal fistulas, and atypical prostatic stromal lesions.

In another embodiment the vector particles of the present invention and medicaments prepared by the use of said particles, respectively can be used for the treatment of diseases being related to the hematopietic system. In this case the pseudotyped vector can be used for selective gene transfer into quiescent hematopietic stem cells (HSC) or precursor cells. With such vectors the therapeutic gene can be transferred and stably inserted into the genome of HSC so that it is present and expressed in any cell type that differentiated from the transduced HSC. For transduction bone marrow cells or leukapheresis cells can be used. Alternatively, with these targeted vectors an *in vivo* application into the patient's bone marrow is possible. Types of diseases that can be treated by this approach include, but are not limited to, monogenetic inherited diseases, like immunodeficiencies as e.g. SCID-X1. In SCID-X1, the pseudotyped vector will transfer the yc-interleukin-receptor gene which is mutated in the patients.

The unique properties of the pseudotyped lentiviral vectors of this invention, i.e. gene delivery into quiescent cells in the G0 phase of the cell cycle, is of special importance for the treatment of many types of neurodegenerative diseases that require the delivery of genes encoding anti-apoptotic or neuro-protective factors into defined subpopulations of neurons. Parkinson's disease e.g., results from the loss of pigmented dopamine-secreting (dopaminergic) cells and subsequent loss of melanin, secreted by the same cells, in the *pars compacta* region of the *substantia nigra.* These neurons project to the striatum and their loss leads to alterations in the activity of the neural circuits within the basal ganglia that regulate movement, in essence an inhibition of the direct pathway and excitation of the indirect pathway. The direct pathway facilitates movement and the indirect pathway inhibits movement, thus the loss of these cells leads to a hypokinetic movement disorder. The lack of dopamine results in increased inhibition of the ventral lateral nucleus of the thalamus, which sends excitatory projections to the motor cortex, thus leading to hypokinesia. Thus, in a further embodiment the vector particles of the present invention and medicaments prepared by the use of said particles, respectively can be used for the treatment of many types of neurodegenerative diseases.

In a further embodiment the pharmaceutical compositions of the present invention are administered alone or in combination with other types of treatment.

### Methods for Monitoring or Tracing the Proliferation, differentiation and/or migration of stem cells

The present pseudotyped vector particles further provide the possibility to trace or monitor the proliferation, differentiation and/or migration of cells. If the gene to be transferred into a cell by the vector particles encodes for a selectable marker, this selectable marker is expressed by the transduced cell and subsequently all progeny cells of the transduced cell. By means of such a selectable marker, the transduced cells as well as any cells derived from these cells (i.e. by proliferation or differentiation) can be detected, for example, using a fluorescently labelled antibody against the selectable marker. As the vector particles of the present invention allow for the transduction of highly undifferentiated cells such as, *inter alia,* stem cells, progenitor cells, neural precursor cells, monocytes and other immune cells, CD34+ cells and CD34+/CD38+ cells residing in the G0 phase of the cell cycle they offer a huge potential for diagnostic and scientific applications.

In a further embodiment the present vector particles are used in a method to identify genes expressed specifically in the G0 phase of the cell cycle. In a preferred embodiment these genes encode for cell surface markers, meaning that the present vector particles are used in a method to identify novel cell surface markers specifically expressed in the G0 phase of the cell cycle.

Such a method for the identification of genes, or preferably cell surface markers, specific for the G0 phase of the cell cycle is based on the properties of the present vector particles compared to, for example, vector particles based on HIV-1. In a first step, an isolated population of cells such as, *inter alia,* CD34⁺ cells is provided that comprises a mixture of cells in the G0, the G1 phase as well as other phases of the cell cycle. Following division of this isolated cell population into two subpopulations, one subpopulation of the cell population will be transduced with at least one selectable marker gene using the present HIV-2 vector particles, while the second subpopulation will be transduced using, for example, vector particles based on HIV-1. Said HIV-1-based vector particles will not be capable of transducing cells residing in the G0 phase of the cell cycle but will transduce cells in the G1 and the S phases of the cell cycle. By contrast, the HIV-2-based vector particles of the present invention will furthermore transduce G0-cells and all other cells. As only those cells in the two separate subpopulations which were transduced by the respective vector particles will express the selectable marker gene, such cells can be isolated by means such as, *inter alia,* FACS or micro beads and consequently subjected to an analysis of the overall mRNA transcripts. Such an mRNA transcript analysis can, *inter alia,* be carried out employing transcriptome analyses utilizing, for example, DNA-CHIP technology. The transcriptome of a cell is defined by all genes expressed by said cell at a given time point and can, for example, be analysed by identification of the RNA, preferable the mRNA, present in a given cell at a given time point. In the next step the transcriptomes of the two subpopulations are compared to identify those transcripts which are specific to cells residing in the G0 phase of the cell cycle. Such transcripts will preferably be found in the subpopulation transduced with the present HIV-2-based vector particles, meaning that a differential analysis of the transcriptomes of the two subpopulations of cells will yield genes preferably expressed during the G0 phase of the cell cycle. By preferably is meant that the transcript of a given gene present in the subpopulation transduced with the present vector particles based on HIV-2 are at least 20%, 30%, 40%, 50%, 60%, 70%, 80%, 90%, 100% or more than 100% more abundant than in the subpopulation transduced with the vector particles based on HIV-1. As some of the G0 phase-specific transcripts will encode cell surface markers, such cell surface markers will likewise be specific for G0 cells. Following the identification of G0 phase-specific cell surface markers antibodies against such markers can be generated by means commonly known to the skilled person. As an example, such antibodies are generated by expression of the identified genes for the production of a recombinant protein which is subsequently used to immunize, for example, a rabbit in order to generate polyclonal antibodies directed against the protein. Additionally, monoclonal antibodies can be generated by means commonly known to the skilled person. Such antibodies can then be used to select viable G0 cells by means such as, *interalia,* FACS. It will be clear that the same method can also be used for the generation of antibodies directed against further proteins specifically expressed during the G0 phase of the cell cycle that are not cell surface markers.

### Examples

### Example 1: Vector Constructs Used for the Generation of Vector Particles

All plasmids used according to the present invention were propagated in *Escherichia coli* strains including Top10F' (Invitrogen BV) or STBL2.

### Vectors Derived from SIVsmmPBj

The *env-*deficient vector construct pPBjDenv was generated by digestion of the infectious molecular clone pPBj1.9 (DEWHURST, S, et al. Sequence analysis and acute pathogenicity of molecularly cloned SIVSMM-PBj14. Nature. 1990, vol.345, no.6276, p.636-640.) with *BstZ17I* at positions 6458 and 7482 and subsequent religation.

The *egfp-*transferring plasmid pPBjDE-EGFP was constructed by ligation of a CMV-promoter/EGFP expression cassette of pEGFP-N1 (Clontech Laboratories Inc, Palo Alto, USA) into the *SnaBI-site* of pPBjDenv.

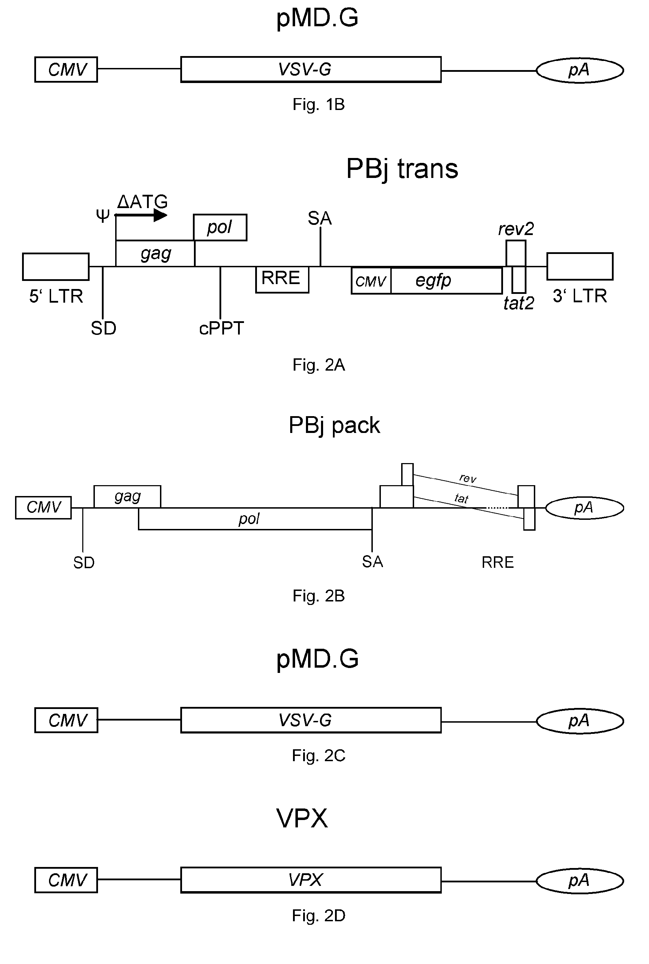

The PBj-derived two-plasmid-system plasmid consists of the plasmids pPBjΔE-EGFP (Fig. 1A) and pMD.G (NALDINI, L, et al. In vivo gene delivery and stable transduction of nondividing cells by a lentiviral vector. Science, 1996, vol.272, no.5259, p.263-7. ; Fig. 1 B). Figure 1 provides a detailed and schematic overview of the PBj-derived two-plasmid system. The plasmid system codes for all accessory proteins including vpx.

The two-plasmid-system of PBj was further progressed to a three-plasmid-system consisting of the transfer-vector (PBj trans), the packaging construct (PBj pack) and pMD.G. For the construction of the transfer-vector the genes coding for Gag. Pol, Vif, Vpx, Vpr, Nef, Tat and Rev were deleted or omitted entirely. The packaging construct was designed to code exclusively for Gag/Pol, Tat and Rev. Figure 2 provides a schematic overview of the PBj-derived three-plasmid-system. Vector particles comprising Vpx can be generated by co-transfection of an expression plasmid coding for Vpx.

### Vectors Derived from HIV-1

To construct the HIV-1-derived plasmid pNL4-3Denv, the *env* reading frame of an infectious HIV-1 molecular clone pNL4-3 (ADACHI, A, et al. Production of acquired immunodeficiency syndrome-associated retrovirus in human and nonhuman cells transfected with an infectious molecular clone. J Virol. 1986, vol.59, no.2, p.284-291.) was deleted by *Acc65ll BsaBI* cleavage at positions 6343 and 7545, digestion by Klenow polymerase, and religation. To generate the HIV-1-derived plasmid pNL4-3DE-EGFP, the CMV/EGFP-expression cassette was amplified by PCR from pEGFP-N1 and inserted into pNL4-3Denv.

The three-plasmid-system used herein is described by Naldini *et al.* (*supra*)

### Vectors Derived from HIV-2

The transfer-vector utilized in the presently disclosed HIV-2 system is based on pSVR, an infectious proviral clone of the ROD strain of HIV-2 containing the replication origin of simian virus 40 (MCCANN, EM, et al. Location of cis-acting signals important for RNA encapsidation in the leader sequence of human immunodeficiency virus type 2. J Virol. 1997, vo1.71, no.5, p.4133-7.). An *Ehe*I fragment (specifically, positions 306 to 5864) was removed from pSVR to generate pSVRΔE. This construct was subsequently digested with *Ns*iI and *Bst*XI*,* deleting a 550-bp fragment of the *env* gene (positions 6369 to 6927) but leaving the Rev-responsive element and the *rev* and *tat* ORFs intact. A DNA linker containing a *Sa*/I site was ligated into this position after blunting with T4 DNA polymerase as described above, to thereby generate pSVR_ENBSaII. The *Ehe*I fragment was reintroduced into this plasmid, yielding pSVRΔNB. Furthermore, in another embodiment a CMVeGFP-cassette was introduced into the *Sal*I restriction sites to thereby generate a transfer vector comprising the heterologous *eGFP* gene.

The packaging construct pHIV-2Δ4 was constructed from pE41 (POESCHLA, E, et al. Identification of a human immunodeficiency virus type 2 (HIV-2) encapsidation determinant and transduction of nondividing human cells by HIV-2-based lentivirus vectors. J Virol. 1998, vol.72, no.8, p.6527-36. , GILBERT, JR, et al. HIV-2 and SIV vector systems. Somat Cell Mol Genet. 2001, vol.26, no.1-6, p.83-98. , CHENG, L, et al. Human immunodeficiency virus type 2 (HIV-2) vector-mediated in vivo gene transfer into adult rabbit retina. Curr Eye Res. 2003, vol.24, no.3, p.196-201.) by a restriction digest using *Bgl*II and *Hind*III to remove the *vif, vpx,* and part of *vpr* accessory genes. The *BsiG*I site within the *nef* coding region was digested and filled in with Klenow and an *Xba*I linker (New-England Biolabs), 5'CTAGTCTAGACTAG-3', was inserted into this particular site to thereby generate a frameshift and translational stop codons in all three of the *nef* reading frames.

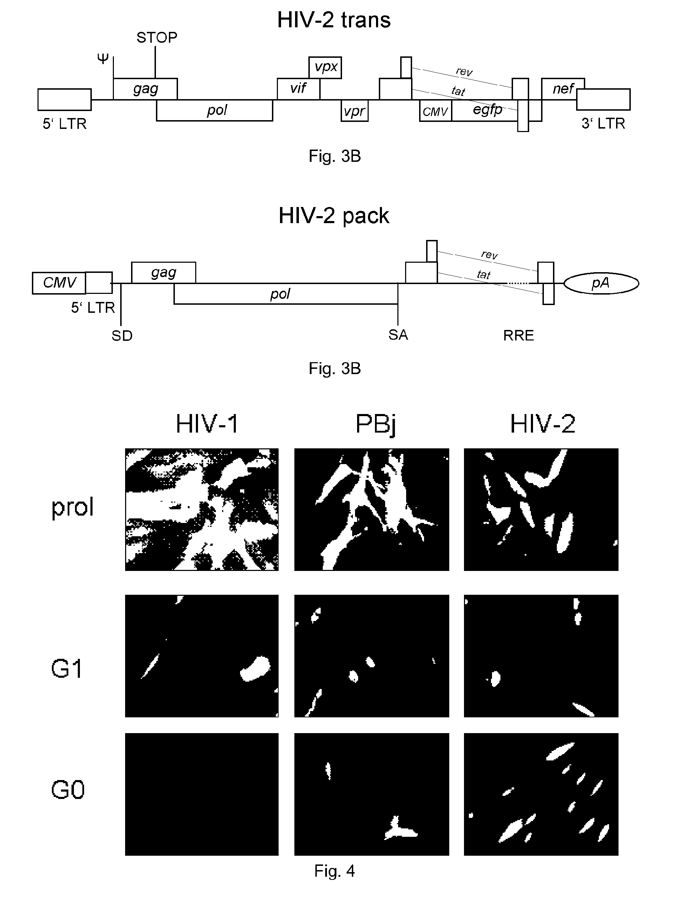

The employed three-plasmid-system of HIV-2 is schematically depicted in Figure 3 and consists of the transfer vector (HIV-2 trans; Figure 3A) coding, *inter alia,* for Vpx, the packaging vector (HIV-2 pack; Figure 3B) and the pMD.G expression vector coding for the envelope VSV-G protein. The gag/pol-expression of transfer-vector of the three-plasmid-system used is abrogated by integrating an additional STOP-codon and the accessory proteins Vpx, Vpr, Vif and Nef are still expressed. The premature stop codon is located in the capsid (CA) region of the gagORF. This construct was generated by digestion of a *Hind*III site (position 1458), subsequent refilling with the Klenow fragment of T4 DNA polymerase, and religation of the DNA.

### Example 2: Generation of Lentiviral Vector Particles

Lentiviral pseudotype vector particles were generated by co-transfection of 293T (ATCC number, SD-3515) cells using LipofectAMINE Plus according to the manufacturer's instructions (Invitrogen Life Technologies) with one of the vector-encoding plasmids, one of the packaging constructs, and pMD.G coding for the VSV-G protein in the case of three-plasmid vector systems.

For two-plasmid systems, only one transfer vector that also encodes for the *gagl pol-*genes is co-transfected with pMD.G. Where *vpx* was complemented, viral vector particles were generated via co-transfection of a further expression plasmid coding for Vpx. Therefore, different expression plasmids have been constructed coding for the Vpx of PBJ (VPXPBJ) or HIV-2 (VPXHIV2).

Vector particle samples containing supernatants were harvested 48 hours following transfection, filtered through 0.45 µm pore-size filters and either stored at -80°C or used directly for transduction. Titration of vectors was performed using human HT1080 cells (ATCC Number CCL-121).

For the transduction of monocytes, vector particles were purified and concentrated by ultracentrifugation of vector-containing supernatants through a 20% sucrose cushion at 25,000 rpm in a Beckman SW28 rotor for 2.0 hrs at 4°C. The pellet was then resuspended in cell culture medium and treated with DNase to get rid of remaining DNA. Subsequently, aliquots were stored at -80°C until use.

### Example 3: Transduction of Primary Alpha-1 Cells

Alpha-1 cells are human diploid fibroblasts, isolated at the "Abteilung Virologie, Insitut für Medizinische Mikrobiologie und Hygiene der Universität Freiburg" and first described in Neumann-Haefelin *et al.* (NEUMANN-HAEFELIN, D, et al. Characterization of a foamy virus isolated from Cercopithecus aethiops lymphoblastoid cells. Med Microbiol Immunol. 1983, vol.172, no.2, p.75-86. They were cultured in DMEM / 10 % FCS /antibiotics / glutamin (mitotic cells) and were used for transduction experiments at passages 12-16 after isolation.

The primary alpha-1 cells which were arrested in the G0 and G1 were incubated for 8 hours with vector-containing supernatants at an MOl of 10 (HIV-2-derived VSV-G pseudotyped vector particles, HIV-1- and PBj-derived VSV-G pseudotyped vector particles were used and generated as described above; the vector particles comprised a transfer vector comprising the eGFP gene). After thorough washing, transduced cells were cultivated for an additional 6 days before analysis of transgene expression either by fluorescence microscopy or by FACS analysis was carried out.

Figure 4 depicts the results of a fluorescence microscopy analysis of the transduced primary alpha-1 cells. As can be clearly see, only the HIV-2-derived VSV-G pseudotyped vector particles were capable of efficiently transducing the primary alpha-1 cells arrested in the G0 phase of the cell cycle.

### Example 4: Isolation and Transduction of Primary Human Monocytes

Human peripheral blood mononuclear cells (PBMCs) were obtained from healthy, anonymous volunteers and purified by Ficoll-Histopaque sedimentation (Sigma diagnostics, St. Louis, USA). Subsequently, samples containing human CD14⁺ monocytes were isolated by negative selection employing the Monocyte Isolation Kit (Miltenyi Biotec, Bergisch-Gladbach, Germany) and magnetic cell sorting (MACS, Miltenyi Biotec) according to the manufacturer's instructions. Cell culture medium (RPMI) contained 10 % autologous donor serum and only 0.5 % FCS. On different days after isolation, monocytes were incubated for 8 hrs with vector-containing supernatants at specific MOl values. After thorough washing, transduced cells were cultivated for an additional 7 days before analysis of transgene expression either by fluorescence microscopy or by FACS analysis was carried out.

The ability of the generated pseudotyped lentiviral vector particles to transduce primary monocytes was evaluated. HIV-2-derived VSV-G pseudotyped vector particles were generated using the two-plasmid-system of HIV-2 as described above and compared to the HIV-1- and PBj-derived VSV-G pseudotyped vector particles, which were likewise generated using the two-plasmid-system. PBMCs were transduced at various time points following their isolation as described above and at an MOl value of 10. The transduced cells were cultivated for 7 days before analysis. The transfer vectors used in the present experiments comprised the heterologous *eGFP* gene in order to facilitate the detection of transduced cells by fluorescence analysis.

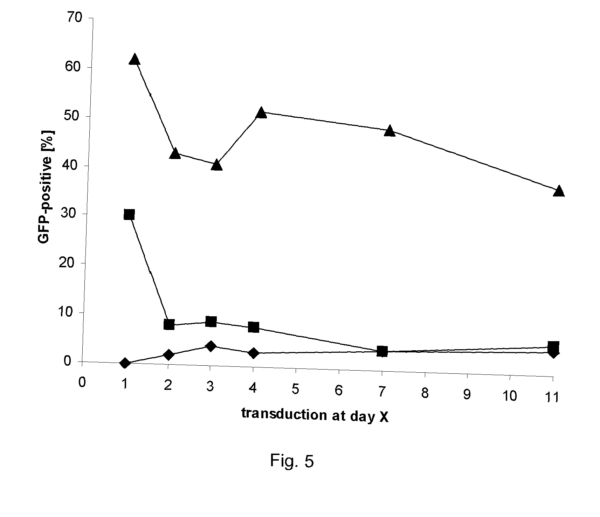

Figure 5 depicts the results of a fluorescence microscopy analysis of the cell transductions at days 1, 2, 3, 4, 7 and 11 following isolation. Clearly, the HIV-2-derived pseudotyped vector particles (▲) were able to transduce the PBMCs in each instance. Surprisingly however, the percentage of GFP-positive (i.e. transduced) cells was markedly increased at each analytical time point compared to those cells transduced with the PBJ-derived (■) and HIV-1-derived (◆) pseudotyped vector particles, respectively. Furthermore and curiously, the value for transduction of PBMCs isolated at day 1 (i.e. transduced at the same day they were isolated) with the HIV-2-derived pseudotyped vector particles is markedly increased compared to the other vector particles. At this particularly early time point almost all of the PBMCs are in the G0 phase of the cell cycle. Specifically, the vector particles of the present invention are especially suitable to transduce cells residing in the G0 phase of the cell cycle.

All results were verified and confirmed by FACS analyses.

The experiments will be repeated with CD34+ cells and CD34+/CD38+ cells and similar results are expected.

Thus, the HIV-2-derived pseudotyped vector particles exhibit an increased and improved transduction efficiency for cells, for example monocytes, stem cells or CD34+ cells or CD34+/CD38+ cells, residing in the G0 phase of the cell cycle.

### Example 5: Transduction of PBMCs and Evaluation of the Transduction Efficiency at Different MOls

The results described in Example 4 clearly show that the HIV-2-derived pseudotyped vector particles of the present invention provide an effective means for the transduction of cells and are thus a promising tool in clinical applications including, *inter alia,* gene therapy. As described above, safety considerations play an important role in the selection of an appropriate vector particle for use in therapeutic or preventive applications, and vector particles that can be employed at a low MOl value are highly preferred since the risk of insertional mutations is reduced or even avoided altogether.

The results provided by Example 4 further indicate that the HIV-2-derived pseudotyped vector particles of the present invention exhibit an extremely high transduction efficiency. These transduction experiments were repeated using decreased MOls of the vector particles of the present invention. Specifically, MOls of 5, 3 and 2 were employed.

The results of these experiments clearly demonstrated that, even at an MOl as low as 2, the HIV-2-derived pseudotyped vector particles of the present invention were capable of efficiently transducing PBMCs. Thus, the pseudotyped vector particles of the present invention can be used at a very low yet safe MOl value in a variety of clinical applications.

### Example 6: Use of the Present HIV-2 Vector Particles for Specific Immunotherapy, Vaccination

The following experiment describes the use of the present HIV-2 vector particles for specific immunotherapy or vaccination, i.e. the present vector particles are utilized as a cellular vaccine. The experiment is set up to investigate the ability of the present HIV-2 vector particles to stimulate cytotoxic T-cells.

Primary monocytes from peripheral blood of healthy human donors as well as CD8+ T cells isolated from the same human donor are isolated and the monocytes are transduced with the present HIV-2 vectors comprising a tumor antigen such as MART that is integrated into the genome of the CD8+ T cells and subsequently expressed by said cells.

The transduced monocytes are subsequently differentiated into dendritic cells (DCs), for example, by stimulation with granulocyte-macrophage colony-stimulating factor (GM-CSF) and interleukin-4 (IL-4) and the transduced mature DCs are incubated with the CD8+ T cells.

The priming of the T cells is then verified via a cytotoxic T cell assay in which the CD8+ T cells are incubated with a melanoma cell line expressing the same tumor antigen (for example MART). The cytotoxic T cell activity is determined by the amount of melanoma cell lysis.

### Example 8: Use of HIV-2 Vector Particles to Monitor Differentiation. Proliferation and/or Localization of Stem Cell Progeny

The following experiments describe the use of the present HIV-2 derived vector particles for the monitoring of the differentiation, proliferation and localization of stem cell progeny.

Hematopoietic stem cells (HSCs) are thought to be a potential source of neural cells for treatment of brain lesions. In order to verify the neurogenic potential of human HSCs, CD34+ HSCs from adult human bone marrow are transduced with a selectable marker gene using the vector particles of the present invention and consequently transplanted into lesions of the developing spinal cord in the chicken embryo. The differentiation, proliferation and localization of said transplanted and transduced human HSCs is then monitored by means of the expressed selectable marker employing techniques as, for example, immunohistochemistry or confocal microscopy. It is expected that the human cells derived from the implanted population will express the neuronal markers NeuN and MAP2, will exhibit neuronal cytoarchitecture and exhibit active membrane properties and spontaneous synaptic potentials characteristic of functionally integrated neurons and will gradually decrease CD34 expression.

Another experiment is based on the findings of Yoon *et al.* (YOON, Y, et al. Clonally expanded novel multipotent stem cells from human bone marrow regenerate myocardium after myocardial infarction. J Clin Invest. 2005, vol.115, no.2, p.326-38.) who identified a subpopulation of stem cells within adult human bone marrow (BM) cells that self-renew without loss of multipotency for more than 140 population doublings and exhibit the capacity for differentiation into cells of all 3 germ layers. Intramyocardial transplantation of said hBMSCs after myocardial infarction resulted in robust engraftment of transplanted cells, which exhibited colocalization with markers of cardiomyocyte (CMC), EC, and smooth muscle cell (SMC) identity, consistent with differentiation of hBMSCs into multiple lineages *in vivo.* Thus, a specific population of multipotent human BM-derived stem cells can induce both therapeutic neovascularization and endogenous and exogenous cardiomyogenesis.

The experimental set-up as described by Yoon *et al.* is repeated, however, the isolated hBMSCs are transduced with a selectable marker utilizing the vector particles of the present invention prior to their transplantation. The differentiation, proliferation and localization of said transplanted and transduced hBMHSCs is then monitored by means of the expressed selectable marker employing techniques as, for example, immunohistochemistry or confocal microscopy.

### References

• ROSS, G, et al. Gene therapy in the United States: a five-year status report. Hum Gene Ther. 1996, vol.7, no. 14, p.1781-90.
• LU, W, et al. Therapeutic dendritic-cell vaccine for chronic HIV-1 infection. Nat Med. 2004, vol.10, no.12, p.1359-65.
• WOODS, NB, et al. Lentiviral vector transduction of NOD/SCID repopulating cells result in multiple vector integrations per transduced cell: risk of insertional mutagenesis. Blood. 2003, vol.101, no.4, p.1284-9.
• GROSZER, M, et al. PTEN negatively regulates neural stem cell self-renewal by modulating G0-G1 cell entry. Proc. Natl. Acad. Sci. U.S.A.. 2006, vol.103, no.1, p.111-6.
• FULTZ, PN, et al. Identification and biologic characterization of an acutely lethal variant of simian immunodeficiency virus from sooty mangabeys (SIV/SMM). AIDS Res Hum Retroviruses. 1989, vol.5, no.4, p.397-409.
• KIRCHHOFF, F, et al. A novel proviral clone of HIV-2: biological and phylogenetic relationship to other primate immunodeficiency viruses. Virology. 1990, vol.177, no.1, p.305-11.
• KIM, VN, et al. Minimal Requirement for a Lentivirus Vector Based on Human Immunodeficiency Virus Type 1. J Virol. 1998, vol.72, p.811-16.
• BURNS, JC, et al. Vesicular stomatitis virus G glycoprotein pseudotyped retroviral vectors: concentration to very high titer and efficient gene transfer into mammalian and nonmammalian cells. Proc. Natl. Acad. Sci. U.S.A.. 1993, vol.90, no.17, p.1833-7.
• MASTROMARINO, P, et al. Characterization of membrane components of the erythrocyte involved in vesicular stomatitis virus attachment and fusion at acidic pH. J Gen Virol. 1998, vol.68, no.9, p.2359-69.
• MARSH, M, et al. Virus entry into animal cells. Adv Virus Res. 1989, vol.107, no.36, p.107-51.
• SAMBROOK, J, et al. Molecular Cloning. A Laboratory Manual. 2nd edition. Edited by SAMBROOK, J, et al. Cold Spring Harbor: Cold Spring Harbor Laboratory, 1989.
• ALTSCHUL, SF, et al. Basic local alignment search tool. J Mol Biol. 1990, vol.215, no.3, p.403-10.
• PEARSON, WR , et al. Improved tools for biological sequence comparison. Proc Natl Acad Sci U S A. 1988, vol.85, no.8, p.2444-8.
• VIGNA, E, et al. Lentiviral vectors: excellent tools for experimental gene transfer and promising candidates for gene therapy. J Gene Med. 2000, vol.2, no.5, p.308-16.
• PALU, G, et al. Progress with retroviral gene vectors. Rev Med Virol. 2000, vol. 10, no.3, p.185-202.
• XU, K, et al. Generation of a stable cell line producing high-titer self-inactivating lentiviral vectors. Mol Ther. 2001, vol.3, no.1, p.97-104.
• GENNARO, AR. Remington's Pharmaceutical Sciences. 18th edition. Edited by GENNARO, AR. Easton, PA: Mack Pub. Co., 1995.
• DEWHURST, S, et al. Sequence analysis and acute pathogenicity of molecularly cloned SIVSMM-PBj14. Nature. 1990, vol.345, no.6276, p.636-640.
• NALDINI, L, et al. In vivo gene delivery and stable transduction of nondividing cells by a lentiviral vector. Science. 1996, vol.272, no.5259, p.263-7.
• ADACHI, A, et al. Production of acquired immunodeficiency syndrome-associated retrovirus in human and nonhuman cells transfected with an infectious molecular clone. J Virol. 1986, vol.59, no.2, p.284-291.
• MCCANN, EM, et al. Location of cis-acting signals important for RNA encapsidation in the leader sequence of human immunodeficiency virus type 2. J Virol. 1997, vol.71, no.5, p.4133-7.
• POESCHLA, E, et al. Identification of a human immunodeficiency virus type 2 (HIV-2) encapsidation determinant and transduction of nondividing human cells by HIV-2-based lentivirus vectors. J Virol. 1998, vol.72, no.8, p.6527-36.
• GILBERT, JR, et al. HIV-2 and SIV vector systems. Somat Cell Mol Genet. 2001, vol.26, no.1-6, p.83-98.
• CHENG, L, et al. Human immunodeficiency virus type 2 (HIV-2) vector-mediated in vivo gene transfer into adult rabbit retina. Curr Eye Res. 2003, vol.24, no.3, p.196-201.
• NEUMANN-HAEFELIN, D, et al. Characterization of a foamy virus isolated from Cercopithecus aethiops lymphoblastoid cells. Med Microbiol Immunol. 1983, vol.172, no.2, p.75-86.
• YOON, Y, et al. Clonally expanded novel multipotent stem cells from human bone marrow regenerate myocardium after myocardial infarction. J Clin Invest. 2005, vol.115, no.2, p.326-38.

## Claims

1. A pseudotyped lentiviral vector particle for the transduction of quiescent cells residing in the G0 phase of the cell cycle, **characterized in that** said vector particle comprises the Gag, Pol and Vpx proteins of HIV-2 or homologs thereof, the G protein of VSV or homologs thereof and an psi-positive RNA molecule that is derived from HIV-2.

2. The pseudotyped lentiviral vector particle according to claim 1, wherein the vector particle further comprises the HIV-2 Vpr, Vif and Nef proteins or homologs thereof.

3. The pseudotyped lentiviral vector particle according to claim 1 or 2, wherein the cells are animal or human cells.

4. The pseudotyped lentiviral vector particle according to anyone of claims 1 through 3, wherein the cells are selected from the group consisting of stem cells, neural stem cells, precursor cells, monocytes, T cells, B cells, CD34+ cells, CD113+ cells and/or CD38+ cells.

5. The pseudotyped lentiviral vector particle according to anyone of claims 1 through 4, wherein the psi-positive RNA molecule is a psi-positive RNA expression vector.

6. The pseudotyped lentiviral vector particle according to claim 5, wherein the psi-positive RNA expression vector comprises at least one gene selected from the group consisting of a selectable marker gene, a gene encoding a fluorescent protein, a gene encoding siRNA, a gene encoding shRNA, a gene encoding an angiogenic factor, a gene encoding a neuro-protective factor, a gene encoding a viral or bacterial antigen, a gene encoding a tumoral antigen, an antigen frame of an infectious agent, a gene encoding an immune-stimulatory factor, and a functional copy of a defective or mutated gene in a patient suffering from an inherited disease.

7. A pharmaceutical composition comprising the pseudotyped lentiviral vector particle according to anyone of claims 1 through 6.

8. The pharmaceutical composition according to claim 7, further comprising a pharmaceutically acceptable carrier.

9. Use of the pseudotyped lentiviral vector according to anyone of claims 1 through 6 for the preparation of a medicament.

10. The use according to claim 9, wherein the medicament is a vaccine.

11. Use of the pseudotyped lentiviral vector according to anyone of claims 1 through 6 for the preparation of a medicament for gene therapy, specific immunotherapy, vaccination or for the treatment or prevention of at least one condition in a subject, wherein the condition is selected from the group consisting of an inherited monogenetic disease, a cardiovascular disease, a neurodegenerative disease, cancer, HIV, Alzheimer disease, Parkinson disease, diabetis, a neuroinflammatory disease, a rheumatic disease, and an autoimmune disease.

12. A method for producing a pseudotyped lentiviral vector particle, the method comprising:
providing a packaging cell line expressing the Gag, Pol and Vpx proteins of HIV-2 or homologs thereof, and the G protein of VSV;
transfecting said packaging cell line with a psi-positive HIV-2 transfer vector; and
allowing the assembly of pseudotyped lentiviral vector particles.

13. The method according to claim 12, wherein the packaging cell line further expresses additional accessory HIV-2 proteins.

14. The method according to claim 13*,* wherein the additional accessory proteins are the HIV-2 Vpr, Vif and Nef proteins or homologs thereof.

15. The method according to anyone of claims 12 through 14, wherein the HIV-2 Vpx, Vpr, Vif and Nef proteins or homologs thereof are encoded by the psi-positive transfer vector, the VSV-G protein or homolog thereof is encoded by a psi-negative expression vector and the packaging cell line is co-transfected with transfer vector and expression vector.

16. The method according to anyone of claims 12 through 15, wherein the psi-positive HIV-2 transfer vector comprises at least one gene selected from the group consisting of a selectable marker gene, a gene encoding a fluorescent protein, a gene encoding siRNA, a gene encoding shRNA, a gene encoding an angiogenic factor, a gene encoding a neuro-protective factor, a gene encoding a viral or bacterial antigen, a gene encoding a tumoral antigen, a gene encoding an immune-stimulatory factor, GFP, eGFP, and a functional copy of a defective or mutated gene in a patient suffering from an inherited disease.

17. The method according to anyone of claims 12 through 14 and 16, wherein the packaging cell line stably expresses one or more of the proteins selected from the group consisting of HIV-2 Gag, Pol, Vpx, Vpr, Vif, Nef and the VSV-G protein.

18. A method of transducing a cell residing in the G0 phase of the cell cycle, the method comprising:
providing a pseudotyped lentiviral vector particle according to anyone of claims 1 through 6; and
contacting said pseudotyped lentiviral vector particle with a cell residing in the G0 phase of the cell cycle.

19. The method according to claim 18, wherein the cell is an animal or a human cell.

20. The method according to claim 18 or 19, wherein the cell is selected from the group consisting of stem cells, neural stem cells, precursor cells, monocytes, T cells, B cells, CD34+ cells, CD113+ cells and/or CD38+ cells.

21. The method according to anyone of claims 18 through 20 wherein the cell is contacted with the pseudotyped lentiviral vector particle at an MOl value of 2.

22. The method according to anyone of claims 18 to 21 for the monitoring of the proliferation, differentiation and/or migration of cells, wherein the gene to be transduced into the cell residing in the G0 phase is a selectable marker and the method further comprises the detection of cells expressing the selectable marker.

23. The method according to claim 21, wherein the marker is GFP or eGFP.

24. A method for the identification of genes expressed specifically during the G0 phase of the cell cycle, the method comprising
(a) providing a population of cells;
(b) dividing the population of cells of step (a) into two subpopulations;
(c) transducing one subpopulation of cells of step (b) with an HIV-1 based vector particle carrying a selectable marker gene and transducing the other subpopulation of cells of step (b) with an HIV-2-based vector particle according to anyone of claims 1 through 6, wherein said HIV-2-based vector particle is further carrying a selectable marker gene;
(d) isolating cells of both subpopulations of step (c) that express the transduced selectable marker gene;
(e) determining the transcriptomes of the cells selected in step (d); and
(f) identifying those genes preferably expressed by the transduced cells of the second subpopulation,
wherein said genes preferably expressed by the transduced cells of the second subpopulation are specifically expressed during the G0 phase of the cell cycle.

25. The method according to claim 24 for the identification of genes coding for cell surface proteins specifically expressed during the G0 phase of the cell cycle.

26. The method according to claims 24 or 25 for the generation of antibodies directed against a protein expressed specifically in the G0 phase of the cell cycle, the method further comprising:
(g) expressing at least one of the genes identified in step (f) to produce a recombinant protein; and
(h) using the recombinant protein to generate antibodies directed against said protein.
